# EUROPEAN PATENT APPLICATION

(11) **EP 4 427 732 A1**
(43) Date of publication of application: **11.09.2024**
(21) Application number: 23160083.4
(22) Date of filing: 06.03.2023
(51) Int. Cl.: A61K 8/65, A61K 8/67, A61K 8/73, A61K 8/9771, A61K 8/9794, A61Q 19/08

(54) **COSMECEUTICAL COMPOSITION**

(71) Applicant: Biodoccle, LLC, Oxnard, CA 93036 (US)
(72) Inventor: Lazzerini, Federica, 8855 Wangen, Schwitz (CH); Clementi, Fabio, 8855 Wangen, Schwitz (CH)
(74) Representative: PGA S.p.A., Milano, Succursale di Lugano

(57) **Abstract**

The present invention relates to a cosmeceutical composition, comprising, relative to the total weight of said composition:
- collagen, in an amount between 620 wt ‰ and 760 wt ‰;
- saline solution, in an amount between 225 wt ‰ and 350 wt ‰;
- hyaluronic acid or a pharmaceutically acceptable salt thereof, in an amount between 1 wt ‰ and 3 wt ‰; and
- at least one natural anti-inflammatory and anti-oxidant agent.

## Description

### FIELD OF THE INVENTION

The present invention relates to the field of compositions for cosmetic/medical applications; in detail, the present invention relates to a cosmeceutical composition for skin restructuration and revitalization.

The present invention further concerns a process for preparing the composition.

### BACKGROUND OF THE INVENTION

Skin revitalization, and in particular skin biorevitalization, is a substantially minimally invasive aesthetic medicine treatment that allows for deeply re-hydrating the skin. Biorevitalization is often applied to the skin of the face, neck, décolleté, and hands.

The purpose of the biorevitalization is to allow the skin to obtain back a young aspect, by means of an enhancement of the cutaneous texture.

Biorevitalization promotes the restoration of the balance of the normal skin physiology, reducing wrinkles and allowing the skin to obtain back firmness.

Biorevitalization treatments typically include subcutaneous micro-injections of hyaluronic acid. In many cases, hyaluronic acid is combined with amino acids and vitamins. The purpose of the micro-injections, which are carried out by means of thin needles, is to re-activate the cutaneous metabolism that in turn promotes re-hydration and firmness to the skin.

Biorevitalization treatments promote the production of elastin (a key component of the extracellular matrix that allows the tissues in the body to resume their shape after stretching and contracting), collagen (the main structural protein of the extracellular matrix) and hyaluronic acid, restoring the elasticity of the fibroblasts and protecting the skin from free radicals that are the main cause of cutaneous ageing.

Skin restructuration, and in particular skin biorestructuration, is a treatment that allows the patient to note a reduction of the micro wrinkles of the skin with a temporary lifting-like effect, a more luminous and compact skin and an enhancement of the micro texture of the skin. Skin biorestructuration process re-activates the normal cellular turnover processes by summing biostimulation and bioregeneration.

Biorestructuration takes place principally through type I heterologous collagen microparticles.

Heterologous collagen is indicated in the regeneration of skin tissue and can be used as an adjuvant in skin biorevitalization by promoting the regeneration of connective tissue in the dermis, creating conditions for the physiological neoformation of collagen.

It finds specific indication in the stimulation of the fibroblastic cellular mechanism, with endogenous collagen neoformation responsible for the development of new fibroblasts and, therefore, new filling tissue.

Such collagen is produced by means of an extraction technique that does not cause modification of the native structure of the protein and does not determine chemical cross-linkings. Collagen thus maintains its triple-helical structure and its sequence of polypeptides chains.

It is known the use of collagen compositions e.g. in form of creams or injections for cosmetic purposes, mainly for the purpose of reducing the wrinkles of a predetermined skin area and in such a way to provide firmness.

Injections are typically performed by trained medical doctors or paramedics, typically in medical offices or day hospital or ambulatory structures.

Applicant has noted that the effects of pure collagen compositions is quite limited.

There is therefore the need of a more effective composition.

### PURPOSES

A purpose of the present invention is to provide a cosmeceutical composition having effect on skin biorevitalization and skin biorestructuration. In particular, purpose of the present invention is to provide a new composition suitable to be injected by means of subcutaneous injections.

A second purpose of the present invention is to provide an easily replicable and safely applicable process for preparing said composition. In particular, the Applicant has provided a process for preparing the composition in such a way to make it possible to distribute it in containers containing a relatively small amount of composition and/or in such a way to make it possible to distribute it in injection-ready containers.

### SUMMARY OF THE INVENTION

The Applicant addressed the problem of providing a more effective composition with respect to available compositions containing pure collagen and suitable to increase skin biorevitalization and skin biorestructuration.

In particular, the Applicant has found that a composition comprising collagen, hyaluronic acid or a pharmaceutically acceptable salt thereof, at least one natural anti-inflammatory and anti-oxidant agent and saline solution is able to reactivate skin metabolism by giving a rehydrated and tightened skin, when injected through micro-infiltrations.

According to the present invention, it is herewith disclosed a cosmeceutical composition, comprising, relative to the total weight of said composition:
- collagen, in an amount between 620 wt ‰ and 760 wt ‰;
- saline solution, in an amount between 225 wt ‰ and 350wt ‰;
- hyaluronic acid or a pharmaceutically acceptable salt thereof, in an amount between 1 wt ‰ and 3 wt ‰; and
- at least one natural anti-inflammatory and anti-oxidant agent.

According to a preferred embodiment, said pharmaceutically acceptable salt of hyaluronic acid is sodium hyaluronate.

According to the invention, the at least one natural anti-inflammatory and anti-oxidant agent is selected from the group consisting of:
- turmeric (Curcuma longa) rhizome extract, and
- Ginkgo Biloba leaf extract, and
- vitamin C.

According to a further non-limiting aspect, turmeric (Curcuma longa) rhizome extract is present in an amount between 2.1 wt ‰ and 3.9 wt ‰, preferably 2.3 wt ‰ and 3.7 wt ‰, even more preferably between 2.4 wt ‰ and 3.5 wt ‰, relative to the total weight of said composition.

Ginkgo Biloba leaf extract is present in an amount between 0.0015 wt ‰ and 0.0080 wt ‰, preferably between 0.0020 wt ‰ and 0.0065 wt ‰, relative to the total weight of said composition.

According to an embodiment, the ratio by weight between said turmeric (Curcuma longa) rhizome extract, and said Ginkgo Biloba leaf extract is between 100:1 and 2000:1, preferably between 200:1 and 1700:1, even more preferably between 300:1 and 1500:1.

Preferably, vitamin C is present in an amount between 5 wt ‰ and 7 wt ‰, preferably in an amount between 5.3 wt ‰ and 6.8 wt ‰ relative to the total weight of said composition.

According to a further non-limiting aspect, the composition further comprises vitamin D, preferably vitamin D3.

Preferably, vitamin D3 is present in an amount between 0.0010 wt ‰ and 0.0080 wt ‰, preferably in an amount between 0.0015 wt ‰ and 0.0075 wt ‰, even more preferably between 0.0020 wt ‰ and 0.0070 wt ‰ relative to the total weight of said composition.

According to a further non-limiting aspect, the composition is an injectable liquid, preferably an injectable-ready liquid. The composition is preferably an injectable-ready composition.

The present invention further refers to a method of manufacturing a cosmeceutical composition, comprising:
a) a step of mixing together:
   - collagen, in an amount between 620 wt ‰ and 760 wt ‰ relative to the total weight of the composition;
   - hyaluronic acid or a pharmaceutically acceptable salt thereof, in an amount between 1 wt ‰ and 3 wt ‰ relative to the total weight of the composition; and
   - at least one natural anti-inflammatory and anti-oxidant agent,
   the step of mixing causing the production of a powder mixture;
b) a step of adding saline solution, in an amount between 225 wt ‰ and 350 wt ‰ relative to the total weight of the composition, to said powder mixture;
c) a step of uniformly mixing the saline solution with the powder mixture, obtaining the cosmeceutical composition.

According to a further non-limiting aspect, the at least one natural anti-inflammatory and anti-oxidant agent according to step a) of the method is selected from the group consisting of:
- turmeric (Curcuma longa) rhizome extract,
- Ginkgo Biloba leaf extract and
- Vitamin C.

According to a further-non limiting aspect, turmeric rhizome extract is added in an amount between 2.1 wt ‰ and 3.9 wt ‰, more preferably 2.3 wt ‰ and 3.7 wt ‰, even more preferably between 2.4 wt ‰ and 3.5 wt ‰, relative to the total weight of said composition.

According to a further-non limiting aspect, Ginkgo Biloba leaf extract is added in an amount between 0.0015 wt ‰ and 0.0080 wt ‰, preferably between 0.0020 wt ‰ and 0.0065 wt ‰ relative to the total weight of said composition.

Turmeric, preferably turmeric (Curcuma longa) rhizome extract, and Ginkgo Biloba leaf extract are mixed in such a way that in the powder mixture the ratio by weight between said turmeric (Curcuma longa) rhizome extract, and said Ginkgo Biloba leaf extract is between 100:1 and 2000:1, preferably between 200:1 and 1700:1, even more preferably between 300:1 and 1500:1.

According to a further non-limiting aspect, said collagen, said hyaluronic acid or a pharmaceutically acceptable salt thereof, and said at least one natural anti-inflammatory and anti-oxidant agent are mixed in a powder form, and/or are in powder and/or fine granular form.

According to a further non-limiting aspect, the step of mixing further includes mixing vitamin D, preferably vitamin D3.

According to a further non-limiting aspect, said vitamin D, preferably vitamin D3, are mixed in a powder form, and/or are in powder and/or fine granular form.

According to a further non-limiting aspect, the method comprises a step of hydrolyzing said collagen.

According to a further non-limiting aspect, the step of hydrolyzing takes place before the step of mixing.

The method may comprise an optional step d) of transferring said liquid composition in a container, preferably a sterile and/or depyrogenated container.

According to a further non-limiting aspect, the method may comprise a step of making said container sterile and/or depyrogenated.

According to a further non-limiting aspect, the method may comprise transferring a single-dose amount of said liquid composition into said container.

According to a further non-limiting aspect, the method may comprise transferring a multi-dose amount of said liquid composition into said container.

According to a further non-limiting aspect, the method may comprise sealing the container with a sealing cap.

According to a further non-limiting aspect, the sealing cap is a pierceable sealing cap, preferably a pierceable, self-closing, cap.

According to a further non-limiting aspect, said cap is a plastic and/or rubber cap.

According to a further non-limiting embodiment, said container is a syringe, preferably a disposable syringe.

### DETAILED DESCRIPTION OF THE INVENTION

The present invention relates to a cosmeceutical composition, comprising, in its simplest form: collagen, hyaluronic acid or a pharmaceutically acceptable salt thereof, saline solution and at least natural anti-inflammatory and anti-oxidant agent.

Object of the present invention is a cosmeceutical composition comprising relative to the total weight of said composition:
- collagen, in an amount between 620 wt ‰ and 760 wt ‰;
- saline solution, in an amount between 225 wt ‰ and 350 wt ‰;
- hyaluronic acid or a pharmaceutically acceptable salt thereof, in an amount between 1 wt ‰ and 3 wt ‰; and
- at least one natural anti-inflammatory and anti-oxidant agent.

The composition according to the invention stimulates the production of elastin, collagen and hyaluronic acid, restores fibroblast elasticity and protects the skin from free radicals (skin aging).

According to a preferred embodiment, the present composition comprises heterologous collagen. Collagen may be extracted from fish, bovine and/or chicken, preferably from costal cartilages of chickens.

According to a particularly preferred embodiment, the present composition comprises hydrolyzed collagen.

Hydrolyzed collagen is obtained by making type I native collagen (undenatured collagen) undergo a process of thermal, acid, alkaline or enzymatic hydrolysis; such process allows to reduce the collagen in protein fragments of a lower molecular weight. By means of the hydrolysis, the long chains of the collagen are reduced in smaller particles. These particles, or fragments, are defined biomimetic since they simulate the degradation action of polymerized collagen, massively stimulate fibroblast and pro-collagen deposit and, consequently, stimulate type III endogenous collagen, substantially eliminating the risk of allergies.

In this way, the behavior of the peptide chains which are part of the collagen is enhanced, and a better cutaneous absorption of said peptide chains is thereby achieved.

At the skin level, hydrolyzed collagen helps to maintain and improve the firmness, tone, and skin turgor while minimizing and improving the presence of wrinkles.

According to a preferred embodiment, said collagen is type I collagen. Applicant notes that anyway said collagen may be, alternatively or in combination, type II and/or type III collagen.

Type I collagen is the collagen that is present in the skin, tendons and bone tissue, and is the collagen type that is most easily recognizable by human immune system. Type I collagen contributes to keep and increase the compactness, tonicity, firmness of the skin, and thus reduces the presence of wrinkles.

Type II collagen may be of a native type and may be suitable to stimulate fibroblasts to create new tissues; thus native collagen may be capable of exerting a relevant biorestructuration activity.

Type III collagen is the second most abundant collagen of soft tissues and dominates early phases of wound healing and granulation tissue formation.

Applicant notes that skin is composed of about 80% of type I collagen, responsible of force and integrity of the skin; the remaining 20% is type III collagen, which is responsible for skin stretching.

The composition according to the invention comprises collagen, in particular hydrolyzed collagen, in an amount between 620 wt ‰ and 760 wt ‰, preferably between 650 wt ‰ and 750 wt ‰ relative to the total weight of the composition.

Hyaluronic acid is an anionic, nonsulfated glycosaminoglycan widely distributed throughout human connective, epithelial, and neural tissues. Advantageously, hyaluronic acid gives the skin its special properties of strength and shape maintenance. Its lack results in a weakening of the skin by promoting the formation of wrinkles and blemishes.

According to a particularly preferred embodiment, the composition according to the invention comprises the sodium salt of hyaluronic acid, sodium hyaluronate.

Sodium hyaluronate is a hydrophilic molecule, with a standing out hygroscopic behavior. A molecule of sodium hyaluronate is capable of retaining up to ten thousands molecules of water. For such reason, it shows moistening properties, absorbs and retains moisture and determines a supply of water in the skin.

According to an embodiment, the composition comprises sodium hyaluronate powder.

Hyaluronic acid or a pharmaceutically acceptable salt thereof is present in an amount between 1 wt ‰ and 3 wt ‰, preferably between 1 wt ‰ and 2.5 ‰ wt relative to the total weight of the composition.

The saline solution, according to a preferred embodiment, may be a 0.9% saline solution of NaCl with distilled water, of pharmaceutical grade. Saline solution is present in an amount between 225 wt ‰ and 350 wt ‰, more preferably between 240 wt ‰ and 330 ‰ wt, relative to the total weight of the composition.

In an embodiment, the sum of hyaluronic acid or a pharmaceutically acceptable salt thereof, saline solution, collagen and anti-oxidant and anti-inflammatory agent reaches the 1000 %o wt.

The at least one natural anti-inflammatory and anti-oxidant agent which is present in the composition according to the invention is selected from the group consisting of turmeric (in particular the extract of the rhizome of the Curcuma longa variety), Ginkgo biloba leaf extract and vitamin C.

Turmeric, and in particular the extract of the rhizome of the Curcuma longa variety, has shown several beneficial properties for the skin. In particular, it has shown anti-wrinkles, anti-flaccidity, anti-inflammatory and/or anti-irritation properties. Moreover, turmeric shows hydrating properties.

The composition according to the invention comprises turmeric (Curcuma longa) rhizome extract in an amount between 2.1 wt ‰ and 3.9 wt ‰, more preferably 2.3 wt ‰ and 3.7 wt ‰, even more preferably between 2.4 ‰ wt and 3.5 wt ‰, relative to the total weight of said composition.

Vitamin C, i.e. the L enantiomer of the ascorbic acid, has several anti-oxidant properties and acts against the skin photo-chromic ageing, effectively protecting the skin against the damages caused by free radicals caused by sun exposure and pollution. At the same time, vitamin C shows anti-wrinkles properties. The brightening effect, determines that dark spots on the skin become lighter, less visible.

When present, vitamin C is present in an amount between 5 wt ‰ and 7 wt ‰, more preferably in an amount between 5.3 wt ‰ and 6.8 wt ‰ relative to the total weight of said composition.

In a preferred embodiment, the cosmeceutical composition comprises vitamin C powder.

Ginkgo Biloba leaf extract helps the elasticity of the skin, by stimulating cell renewing. Therefore, it acts against skin ageing.

Ginkgo Biloba leaf extract contains many different flavone glycosides and terpenoids.

In particular, Ginkgo Biloba leaf extract has anti-inflammatory and anti-oxidant properties and increases blood microcirculation and skin oxygenation. Ginkgo Biloba leaf extract shows effects against free radicals that produces beneficial effects to the cell metabolism, and prevents lipid peroxidation of the membranes.

Ginkgo Biloba leaf extract shows beneficial effects against the reactive forms of the oxygen (ROS), sustaining the normal function of fibroblasts.

Ginkgo Biloba leaf extract has been mainly selected for the anti-inflammatory and anti-oxidant properties; it may be selected as an additional ingredient due to the increase in microcirculation and skin oxygenation.

According to the invention, the composition comprises Ginkgo biloba leaf extract powder and/or turmeric rhizome extract powder.

In the composition according to the invention, Ginkgo Biloba leaf extract powder may be used as an alternative to the turmeric rhizome extract powder or as a further additive element, in an amount between 0.0015 wt ‰ and 0.0080 wt ‰, preferably between 0.0020 wt ‰ and 0.0065 wt ‰, relative to the total weight of the composition.

In particular, turmeric rhizome extract powder and Ginkgo Biloba leaf extract powder are combined with a ratio by weight between 100:1 and 2000:1, preferably between 200:1 and 1700:1, even more preferably between 300:1 and 1500:1. As it will be apparent by further reading the present description, example 1 of the composition is provided with turmeric rhizome extract and Ginkgo Biloba leaf extract in a ratio by weight of 1000:1.

Preferably, the anti-oxidant and anti-inflammatory agent is turmeric rhizome extract and/or vitamin C.

In addition to the aforementioned components, the Applicant has found that several other elements may be beneficially combined thereto. In particular, the composition according to the invention may further comprise vitamin D, and in particular D3. Vitamin D has a fundamental role in the skin barrier and in the growth of skin cells, and sustains local immune system.

When present, vitamin D, preferably vitamin D3, is present in an amount between 0.0010 wt ‰ and 0.0080 wt ‰; preferably between 0.0015 wt ‰ and 0.0075 wt ‰, more preferably between 0.0020 and 0.0070 wt ‰ relative to the total weight of said composition.

The composition herein disclosed is a liquid composition that may be injected subcutaneously by means of very small needles; the use of very small needles causes less discomfort in the patient during the treatment.

In a preferred embodiment, the composition comprises relative to the total weight of said composition:
- collagen, preferably hydrolyzed collagen, in an amount between 650 wt ‰ and 750 wt ‰
- saline solution in an amount between 240 wt ‰ and 330 ‰ wt;
- hyaluronic acid or a pharmaceutically acceptable salt thereof in an amount between 1 wt ‰ and 2.5 wt ‰;
- turmeric (Curcuma longa) rhizome extract in an amount between 2.4 wt ‰ and 3.5 wt %o;
- Ginkgo biloba leaf extract in an amount between 0.0020 ‰ wt and 0.0065 wt ‰.
- vitamin C in an amount between 5.3 wt ‰ and 6.8 wt ‰; and
- vitamin D, preferably vitamin D3, in an amount of 0.0020 wt ‰ and 0.0070 wt ‰.

In an embodiment, the composition comprises collagen, preferably hydrolyzed collagen, saline solution, hyaluronic acid or a pharmaceutically acceptable salt thereof, turmeric (Curcuma longa) rhizome extract, vitamin C, and vitamin D, preferably vitamin D3.

In another embodiment, the composition consist of hydrolyzed collagen, hyaluronic acid or a pharmaceutically acceptable salt thereof, saline solution, turmeric (Curcuma longa) rhizome extract, vitamin C, and vitamin D3.

In another embodiment, the composition consist of hydrolyzed collagen, hyaluronic acid or a pharmaceutically acceptable salt thereof, saline solution, turmeric (Curcuma longa) rhizome extract, Gingko Biloba leaf extract, vitamin C, and vitamin D3.

The composition according to the invention may comprise one or more cosmeceutical excipients, wherein said excipient refers to any compound that is acceptable in the sense of being compatible with the other ingredients of the composition.

The present invention further refers to a method of manufacturing a cosmeceutical composition, comprising relative to the total weight of said composition:
a) a step of mixing together:
   - collagen, in an amount between 620 wt ‰ and 760 wt ‰ relative to the total weight of the composition;
   - hyaluronic acid or a pharmaceutically acceptable salt thereof, in an amount between 1 wt ‰ and 3 wt ‰ relative to the total weight of the composition; and
   - at least one natural anti-inflammatory and anti-oxidant agent,
   the step of mixing causing the production of a powder mixture;
b) a step of adding saline solution to said powder mixture, in an amount between 225 wt ‰ and 350 wt ‰ relative to the total weight of the composition;
c) a step of uniformly mixing the saline solution with the powder mixture, obtaining the cosmeceutical composition..

The at least one natural anti-inflammatory and anti-oxidant agent is selected from the group consisting of turmeric (Curcuma longa) rhizome extract, vitamin C and Ginkgo Biloba leaf extract.

Preferably, turmeric rhizome extract is added in an amount between 2.1 wt ‰ and 3.9 wt ‰, preferably between 2.3 wt ‰ and 3.7 wt ‰, even more preferably between 2.4 wt %o and 3.5 wt %o, relative to the total weight of the composition, and/or Ginkgo Biloba leaf extract is added in an amount between 0.0015 wt ‰ and 0.0080 wt ‰, preferably between 0.0020 wt ‰ and 0.0065 wt ‰ relative to the total weight of the composition, and/or vitamin C is added in an amount between 5 wt ‰ and 7 wt ‰, preferably in an amount between 5.3 wt ‰ and 6.8 wt ‰ relative to the total weight of the composition.

The method may further include the step of mixing vitamin D, preferably vitamin D3, together with the components mixed in step a).

According to this embodiment, vitamin D, preferably vitamin D3, is mixed in an amount between 0.0010 wt ‰ and 0.0080 wt ‰, preferably in an amount between 0.0015 wt ‰ and 0.0075 wt ‰, even more preferably between 0.0020 wt ‰ and 0.0070 wt ‰ relative to the total weight of the composition.

The composition as above defined may be advantageously used for skin restructuration and revitalization, in particular for facial skin, neck, décolleté, and hands restructuration and revitalization.

In particular, the composition above described may be used in a method for skin biorevitalization and biorestructuration comprising performing subcutaneous microinjections of the composition above described to a subject in need thereof.

Said subcutaneous microinjections may be carried out preferably at a distance of 0.5 to 5 cm one another, preferably at a distance of 1 to 3 cm, even more preferably at a distance of 1 cm one another.

In particular, before said subcutaneous microinjections, the subject in need thereof may be subjected to anesthesia, for example local anesthesia.

Said subcutaneous microinjections of the composition according to the invention may be preferably carried out on facial skin, neck, décolleté, and hands.

The treatment may be carried out 4 or 5 times, at intervals of about 1 month; following the first 4 or 5 treatments, a sustainment treatment may be further carried out with the same procedure at an interval of about 6 months. Should the patient so request, a pretreatment with local anesthetic may be carried out. Applicant has observed that the composition herewith disclosed is compatible with the traditional anesthetic skin creams without side effects or adverse interactions.

### EXAMPLES

Hereinafter, some embodiments of the composition according to the invention are provided.

The hydrolyzed collagen used for the compositions below is the product commercialized under the trademark name Glow25^{®}.

### Example 1

A first and preferred example of the composition comprises:
10000 mg of hydrolyzed collagen Glow25@
25 mg of hyaluronic acid
40 mg of turmeric (Curcuma longa) rhizome extract
80 mg of vitamin C
50 µg of vitamin D3
40 µg of Ginkgo Biloba leaf extract
3854.91 mg of saline solution

In particular, the saline solution is a 0.9% saline solution of NaCl with distilled water.

The above amounts allow obtaining a 14g injectable composition.

If the above amounts are provided in terms of ‰ by weight, it is clear that the first and preferred example of the composition comprises:
714.29 ‰ of hydrolyzed collagen Glow25@
1.786 ‰ of hyaluronic acid
2.857 ‰ of turmeric (Curcuma longa) rhizome extract
5.714 ‰ of vitamin C
0.0035714 ‰ of vitamin D3
0.0028572 ‰ of Ginkgo Biloba leaf extract
275.35 ‰ of saline solution

In the first example, hyaluronic acid is in the form of sodium hyaluronate powder.

### Example 2

A second example of the composition comprises:
10000 mg of hydrolyzed collagen Glow25@
25 mg of hyaluronic acid
40 mg of turmeric (Curcuma longa) rhizome extract
80.05 mg of vitamin C
40 µg of vitamin D3
3854.91 mg of saline solution

In particular, the saline solution is a 0.9% saline solution of NaCl with distilled water.

The above amounts allow obtaining a 14g injectable composition.

If the above amounts are provided in terms of ‰ by weight, it is clear that the first and preferred example of the composition comprises:
714.29 ‰ of hydrolyzed collagen Glow25@
1.786 ‰ of hyaluronic acid
2.857 ‰ of turmeric (Curcuma longa) rhizome extract
5.718 ‰ of vitamin C
0.0028572 ‰ of vitamin D3
275.35 ‰ of saline solution

### Example 3

A third example of the composition comprises:
10000 mg of hydrolyzed collagen Glow25@
25 mg of hyaluronic acid
40 mg of turmeric (Curcuma longa) rhizome extract
80 mg of vitamin C
90 µg of vitamin D3
3854.91 mg of saline solution

In particular, the saline solution is a 0.9% saline solution of NaCl with distilled water.

The above amounts allow obtaining a 14g injectable composition.

If the above amounts are provided in terms of ‰ by weight, it is clear that the first and preferred example of the composition comprises:
714.29 ‰ of hydrolyzed collagen Glow25@
1.786 ‰ of hyaluronic acid
2.857 ‰ of turmeric (Curcuma longa) rhizome extract
5.714 ‰ of vitamin C
0.0064286 ‰ of vitamin D3
275.35 ‰ of saline solution

### Example 4

A fourth example of the composition comprises:
10000 mg of hydrolyzed collagen Glow25@
25 mg of hyaluronic acid
40 mg of turmeric (Curcuma longa) rhizome extract
80 mg of vitamin C
90 µg of Ginkgo Biloba leaf extract
3854.91 mg of saline solution

In particular, the saline solution is a 0.9% saline solution of NaCl with distilled water.

The above amounts allow obtaining a 14g injectable composition.

If the above amounts are provided in terms of ‰ by weight, it is clear that the first and preferred example of the composition comprises:
714.29 ‰ of hydrolyzed collagen Glow25@
1.786 ‰ of hyaluronic acid
2.857 ‰ of turmeric (Curcuma longa) rhizome extract
5.714 ‰ of vitamin C
0.0064286 ‰ of Ginkgo Biloba leaf extract
275.35 ‰ of saline solution

In the fourth example, hyaluronic acid is in the form of sodium hyaluronate powder.

### Example 5

A fifth example of the composition comprises:
9992 mg of hydrolyzed collagen Glow25@
20 mg of hyaluronic acid
85 mg of vitamin C
50 µg of vitamin D3
40 µg of Ginkgo Biloba leaf extract
3902.91 mg of saline solution

In particular, the saline solution is a 0.9% saline solution of NaCl with distilled water.

The above amounts allow obtaining a 14g injectable composition.

If the above amounts are provided in terms of ‰ by weight, it is clear that the first and preferred example of the composition comprises:
713.71 ‰ of hydrolyzed collagen Glow25@
1.429 ‰ of hyaluronic acid
6.071 ‰ of vitamin C
0.0035714 ‰ of vitamin D3
0.0028572 ‰ of Ginkgo Biloba leaf extract
278.77 ‰ of saline solution

In the fifth example, hyaluronic acid is in the form of sodium hyaluronate powder.

### Example 6

A sixth example of the composition comprises:
8000 mg of hydrolyzed collagen Glow25@
25 mg of hyaluronic acid
40 mg of turmeric (Curcuma longa) rhizome extract
80 mg of vitamin C
50 µg of vitamin D3
40 µg of Ginkgo Biloba leaf extract
3854.91 mg of saline solution

In particular, the saline solution is a 0.9% saline solution of NaCl with distilled water.

The above amounts allow obtaining a 12g injectable composition.

If the above amounts are provided in terms of ‰ by weight, it is clear that the first and preferred example of the composition comprises:
667 ‰ of hydrolyzed collagen Glow25@
2.083 ‰ of hyaluronic acid
3.333 ‰ of turmeric (Curcuma longa) rhizome extract
6.666 ‰ of vitamin C
0.0041666 ‰ of vitamin D3
0.0033333 ‰ of Ginkgo Biloba leaf extract
321.24 ‰ of saline solution

In the sixth example, hyaluronic acid is in the form of sodium hyaluronate powder.

### Example 7

A seventh example of the composition comprises:
11500 mg of hydrolyzed collagen Glow25@
25 mg of hyaluronic acid
40 mg of turmeric (Curcuma longa) rhizome extract
80 mg of vitamin C
40 µg of vitamin D3
50 µg of Ginkgo Biloba leaf extract
3900 mg of saline solution

In particular, the saline solution is a 0.9% saline solution of NaCl with distilled water.

The above amounts allow obtaining a 15.5451 g injectable composition.

If the above amounts are provided in terms of ‰ by weight, it is clear that the first and preferred example of the composition comprises:
739.8 ‰ of hydrolyzed collagen Glow25@
1.608 ‰ of hyaluronic acid
2.573 ‰ of turmeric (Curcuma longa) rhizome extract
5.146 ‰ of vitamin C
0.002573 ‰ of vitamin D3
0.003216 ‰ of Ginkgo Biloba leaf extract
250.88 ‰ of saline solution

In the seventh example, hyaluronic acid is in the form of sodium hyaluronate powder.

### Example 8

A eighth example of the composition comprises:
10000 mg of hydrolyzed collagen Glow25@
25 mg of hyaluronic acid
40 mg of turmeric (Curcuma longa) rhizome extract
50 µg of vitamin D3
30 µg of Ginkgo Biloba leaf extract
3934 mg of saline solution

In particular, the saline solution is a 0.9% saline solution of NaCl with distilled water.

The above amounts allow obtaining a 14g injectable composition.

If the above amounts are provided in terms of ‰ by weight, it is clear that the first and preferred example of the composition comprises:
714.3 ‰ of hydrolyzed collagen Glow25@
1.785 ‰ of hyaluronic acid
2.857 ‰ of turmeric (Curcuma longa) rhizome extract
0.003571 ‰ of vitamin D3
0.002142 ‰ of Ginkgo Biloba leaf extract
281.065 ‰ of saline solution.

In the eighth example, hyaluronic acid is in the form of sodium hyaluronate powder.

It is herewith disclosed a manufacturing process for the aforementioned composition.

Hydrolyzed collagen, hyaluronic acid or a pharmaceutically acceptable salt thereof, - at least one between the Vitamin C, turmeric rhizome extract, and Ginkgo Biloba leaf extract and vitamin D (when present) are provided in pharmaceutical grade, pure, powders or fine granules.

The aforementioned elements are mixed together in a mixer by means of any between a substantially automated process or a manual process, up to the moment at which a powder mixture is obtained.

Subsequently the method comprises the addition of saline solution, in an amount sufficient to obtain the above-disclosed ranges or precise amounts. The addition of saline solution determines obtaining, at first a non-uniform fluid mixture that is subsequently mixed again.

A further subsequent mixing step therefore takes place, for obtaining a substantially uniform liquid that corresponds to the cosmeceutical composition, with no significant solid residual part that may clog the needle of a syringe. It may be noted that the composition can be injected through needles with gauges down to 33 or 34; solid residual parts may therefore be carefully avoided.

The so obtained liquid final cosmeceutical composition is then optionally filtered and introduced, preferably in a controlled atmosphere environment, in containers. It is not necessary to use light-attenuating (e.g. brown, green) glass or plastic vessels.

The composition so obtained may be introduced in a sterile container.

In an embodiment, which is non-limiting, the container is an injection-ready container. An injection-ready container may be provided with a sealing stopper, which is preferably of a self-sealing type. The stopper is pierceable, and in particular is configured and specifically destined to be pierced by a needle, in particular by a needle of a syringe.

For the purposes of the present invention, for "self-sealing" shall be intended a cap that, once pierced by a needle, in particular a needle of a syringe, and once such needle is extracted, in particular fully extracted, by said cap, closes automatically without any substantial intervention of a user.

In particular a plastic and/or rubber stopper, suitable to allow the penetration of the needle of a syringe without subsequently spilling any residual liquid that may be present in the composition. The container may be a single-dose vial or a multi-dose vial. The container may be preferably a depyrogenated container.

Thus, in an embodiment the method comprises collecting a single-dose amount of the liquid final composition into the container. In an alternative embodiment the method comprises collecting a multi-dose amount of the liquid final composition into the container.

A single-dose amount of the liquid final composition may be, for instance, a dose sufficient to fill substantially a 0.5 ml syringe, or a 1 ml syringe, or a 2 ml syringe, or a 3 ml syringe or a 5 ml syringe.

A multi-dose amount of the liquid final composition may be, for instance, an amount of 10ml or more.

In an embodiment, such container is a syringe, in particular a disposable syringe. This allows the medical doctor or any practitioner to implement the treatment without performing the action of perforating the pierceable sealing cap of the container with an additional syringe.

The advantages of the composition herewith disclosed are clear in view of the above description. The composition is substantially natural, has proven to be well tolerated also by sensitive patients; the composition, when injected, determines limited side effects as swelling or bleeding or itching. The composition substantially determines an immediate, prolonged, and effective firmness of the skin. In particular, when Ginkgo Biloba leaf extract is present, a temporary effect of redness in the skin is the result of a relevant oxygenation of the skin in the area surrounding the injection site.

The herewith disclosed composition is easy to manufacture and may be produced in a cost-effective way.

Several adaptations or additions can be carried out to the object of the present disclosure without for this departing from the scope of protection provided by the annexed claims.

## Claims

1. A cosmeceutical composition, comprising, relative to the total weight of said composition:
- collagen, in an amount between 620 wt ‰ and 760 wt ‰;
- saline solution, in an amount between 225 wt ‰ and 350 wt ‰;
- hyaluronic acid or a pharmaceutically acceptable salt thereof, in an amount between 1 wt ‰ and 3 wt ‰; and
- at least one natural anti-inflammatory and anti-oxidant agent.

2. The composition according to claim 1, wherein the at least one natural anti-inflammatory and anti-oxidant agent is selected from the group consisting of:
- turmeric (Curcuma longa) rhizome extract,
- Ginkgo Biloba leaf extract, and
- vitamin C.

3. The composition according to claim 2, wherein the turmeric rhizome extract is present in an amount between 2.1 wt ‰ and 3.9 wt ‰, preferably 2.3 wt ‰ and 3.7 wt ‰, more preferably between 2.4 wt ‰ and 3.5 wt ‰ relative to the total weight of the composition.

4. The composition according to claim 2, wherein said Ginkgo Biloba leaf extract is present in an amount between 0.0015 wt ‰ and 0.0080 wt ‰, preferably between 0.0020 wt ‰ and 0.0065 wt ‰, relative to the total weight of the composition.

5. The composition according to any claim 2 to 4, wherein the ratio by weight between said turmeric rhizome extract and said Ginkgo Biloba leaf extract is between 100:1 and 2000:1, preferably between 200:1 and 1700:1, even more preferably between 300:1 and 1500:1.

6. The composition according to any claim 2 to 4, wherein said vitamin C is present in an amount between 5 wt ‰ and 7 wt ‰, preferably in an amount between 5.3 wt ‰ and 6.8 wt ‰ relative to the total weight of the composition.

7. The composition according to one or more of the preceding claims, further comprising vitamin D, preferably vitamin D3.

8. The composition according to claim 7, wherein said vitamin D is in an amount between 0.0010 wt ‰ and 0.0080 wt ‰, preferably in an amount between 0.0015 wt ‰ and 0.0075 wt ‰, even more preferably between 0.0020 wt ‰ and 0.0070 wt ‰ relative to the total weight of the composition.

9. The composition according to one or more of the preceding claims, wherein said hyaluronic acid or a pharmaceutically acceptable salt thereof is in an amount between 1 wt ‰ and 2.5 wt ‰ relative to the total weight of the composition.

10. The composition according to one or more of the preceding claims, wherein said collagen is hydrolyzed collagen, preferably type I hydrolyzed collagen.

11. The composition according to one or more of the preceding claims, wherein said collagen, preferably hydrolyzed collagen is in an amount between 650 wt ‰ and 750 wt ‰ relative to the total weight of the composition.

12. The composition according to one or more of the preceding claims, comprising relative to the total weight of said composition:
- collagen, preferably hydrolyzed collagen, in an amount between 650 wt ‰ and 750 wt %o;
- saline solution in an amount between 240 wt ‰ and 330 ‰ wt;
- hyaluronic acid or a pharmaceutically acceptable salt thereof in an amount between 1 wt ‰ and 2.5 wt ‰;
- turmeric (Curcuma longa) rhizome extract in an amount between 2.4 wt ‰ and 3.5 wt %o;
- Ginkgo biloba leaf extract in an amount between 0.0020 ‰ wt and 0.0065 wt ‰.
- vitamin C in an amount between 5.3 wt ‰ and 6.8 wt ‰; and
- vitamin D, preferably vitamin D3, in an amount of 0.0020 wt ‰ and 0.0070 wt ‰.

13. A method of manufacturing the cosmeceutical composition according to any one of claims 1 to 12, comprising:
a) a step of mixing together:
- collagen, in an amount between 620 wt ‰ and 760 wt ‰ relative to the total weight of the composition;
- hyaluronic acid or a pharmaceutically acceptable salt thereof, in an amount between 1 wt ‰ and 3 wt ‰ relative to the total weight of the composition; and
- at least one natural anti-inflammatory and anti-oxidant agent,
the step of mixing causing the production of a powder mixture;
b) a step of adding a saline solution, in an amount between 225 wt ‰ and 350 wt ‰ relative to the total weight of the composition, to said powder mixture obtained according to step a);
c) a step of uniformly mixing the saline solution with the powder mixture, obtaining the cosmeceutical composition.

14. The method according to claim 13, wherein the anti-inflammatory and anti-oxidant agent according to step a) is selected from the group consisting of:
- turmeric (Curcuma longa) rhizome extract,
- Ginkgo Biloba leaf extract and
- vitamin C,
wherein turmeric rhizome extract is preferably added in an amount between 2.1 wt ‰ and 3.9 wt ‰, more preferably 2.3 wt ‰ and 3.7 wt ‰, even more preferably between 2.4 wt and 3.5 ‰ wt relative to the total weight of the composition, and/or wherein Ginkgo Biloba leaf extract is preferably added in an amount between 0.0015 wt ‰ and 0.0080 wt ‰, more preferably between 0.0020 wt ‰ and 0.0065 wt ‰, relative to the total weight of the composition, and/or vitamin C is preferably added in an amount between 5 wt ‰ and 7 wt ‰, more preferably in an amount between 5.3 wt ‰ and 6.8 wt ‰ relative to the total weight of the composition.

15. The method according to claim 13 or 14, wherein the step a) of mixing further includes mixing vitamin D, preferably vitamin D3, in an amount between 0.0010 wt ‰ and 0.0080 wt ‰, preferably in an amount between 0.0015 wt ‰ and 0.0075 wt ‰, even more preferably between 0.0020 wt ‰ and 0.0070 wt ‰ relative to the total weight of the composition.

16. The method according to one or more of claims 13 to 15, comprising a step of hydrolyzing said collagen.
